# EUROPEAN PATENT APPLICATION

(11) **EP 4 755 336 A1**
(43) Date of publication of application: **10.06.2026**
(21) Application number: 25221291.5
(22) Date of filing: 05.12.2025
(51) Int. Cl.: A61B 34/10, A61B 34/20

(54) **ADJUSTMENT OR DISPLAY OF OPTIONS OF POSITIONAL OR ORIENTATION IMPLICATIONS ON SURGICAL TOOL USAGE**

(30) Priority: 06.12.2024 US 202418971908
(71) Applicant: Cilag GmbH International, 6300 Zug (CH)
(72) Inventor: SHELTON, IV,, Frederick E., Cincinnati, 45242 (US); HARRIS,, Jason L., Cincinnati, 45242 (US); DECK,, Andrew C., Cincinnati, 45242 (US)
(74) Representative: Carpmaels & Ransford LLP

(57) **Abstract**

A surgical smart system may configure positioning and orientation of surgical instruments during surgical procedures by considering current and subsequent surgical steps. The system may include a processor configured to identify an active surgical step and a subsequent surgical step associated with a surgical instrument. The system may determine multiple possible positional configurations for the active step, assess the instrument's and patient's orientations, and calculate corresponding downstream configurations for the subsequent step. Based on the determination, the system may adjust the current configuration and select a suitable configuration for the active step.

## Description

### BACKGROUND

This application is related to the following, filed contemporaneously, the contents of each of which are incorporated by reference herein:
- Attorney Docket No. END9638USNP1, entitled PROGRESSIVE ADVANCEMENT OF AUTOMATED LEVEL BASED ON LEARNED COMPLIMENTARY ASSISTANCE
- Attorney Docket No. END9638USNP2, entitled ADJUSTING AUTOMATED COOPERATIVE OPERATIONS BASED ON SITUATIONALLY DERIVED CONSTRAINTS,
- Attorney Docket No. END9638USNP3, entitled ASSISTANCE ADVANCEMENT MULTI-SYSTEM INTERACTION,
- Attorney Docket No. END9638USNP4, entitled MONITORING AND IDENTIFYING SURGEON CONTROL AND SUGGESTING A TASK THAT MAY BE DONE AUTONOMOUSLY,
- Attorney Docket No. END9638USNP5, entitled CONTROL OF INFORMATION FLOW, PRIORITIZATION AND MANIFESTATION OF DATA ASSOCIATED WITH AN ACTIVE HCP INTERACTION SPACE,
- Attorney Docket No. END9638USNP6, entitled ADAPTIVE RETRACTION FORCE CONTROL, and
- Attorney Docket No. END9638USNP8, entitled ADJUSTMENT OF PHYSIOLOGIC FUNCTION SUPPLEMENTATION CONTROL.

The contents of each of the following are incorporated by reference herein:
- U.S. Patent Application No. 18/810,323 entitled METHOD FOR MULTI-SYSTEM INTERACTION, filed on August 20, 2024;
- U.S. Patent Application No. 18/960,006 entitled METHOD FOR SMART SURGICAL SYSTEMS filed on November 26, 2024; and
- U.S. Patent Application No. 18/954,186 entitled METHOD FOR MULTI-SYSTEM INTERACTION, filed on November 20, 2024.

### BACKGROUND

Surgical procedures are typically performed in surgical operating theaters or rooms in a healthcare facility such as, for example, a hospital. Various surgical devices and systems are utilized in performance of a surgical procedure. In the digital and information age, medical systems and facilities are often slower to implement systems or procedures utilizing newer and improved technologies due to patient safety and a general desire for maintaining traditional practices.

### SUMMARY

A surgical smart system may configure positioning and orientation of surgical instruments during surgical procedures by considering current and subsequent surgical steps. The system may include a processor configured to identify an active surgical step and a subsequent surgical step associated with a surgical instrument. The system may determine multiple possible positional configurations for the active step, assess the instrument's and patient's orientations, and calculate corresponding downstream configurations for the subsequent step. Based on the determination, the system may adjust the current configuration and select a suitable configuration for the active step.

The system may determine anticipated effects of positional and orientation configurations on anatomical structures. The system may generate display notifications to present the anticipated effects and allow the user to make a decision. The system may receive input based on the notifications to select the preferred configuration and may generate control signals to maneuver the surgical instrument accordingly.

The system may address limitations by identifying unviable configurations due to factors such as reach limitations, articulation range, or port placement. The system may adjust the available options accordingly and notify the user of the causes behind an unviable configuration. By analyzing elements such as trocar placement relative to patient and instrument orientation, the system may calculate access limitations and propose alternative configurations to overcome the limitation.

### BRIEF DESCRIPTION OF THE DRAWINGS

FIG. 1 is a block diagram of a computer-implemented surgical system.
FIG. 2 shows an example surgical system in a surgical operating room.
FIG. 3 illustrates an example surgical hub paired with various systems.
FIG. 4 shows an example situationally aware surgical system.
FIG. 5 illustrates an example surgical system that may include a surgical instrument.
FIG. 6A illustrates an example surgical setup including a first trocar and a second trocar used in part of a surgical procedure.
FIG. 6B illustrates an example surgical setup including a first trocar and a second trocar used in part of a surgical procedure.
FIG. 7 illustrates an example flowchart associated with the operation of a system (e.g., a smart system/surgical smart system).
FIG. 8 illustrates an example artificial intelligence/machine learning flowchart associated with how the system may receive input, process data, and produce output related to a surgical procedure.

### DETAILED DESCRIPTION

A more detailed understanding may be had from the following description, given by way of example in conjunction with the accompanying drawings.

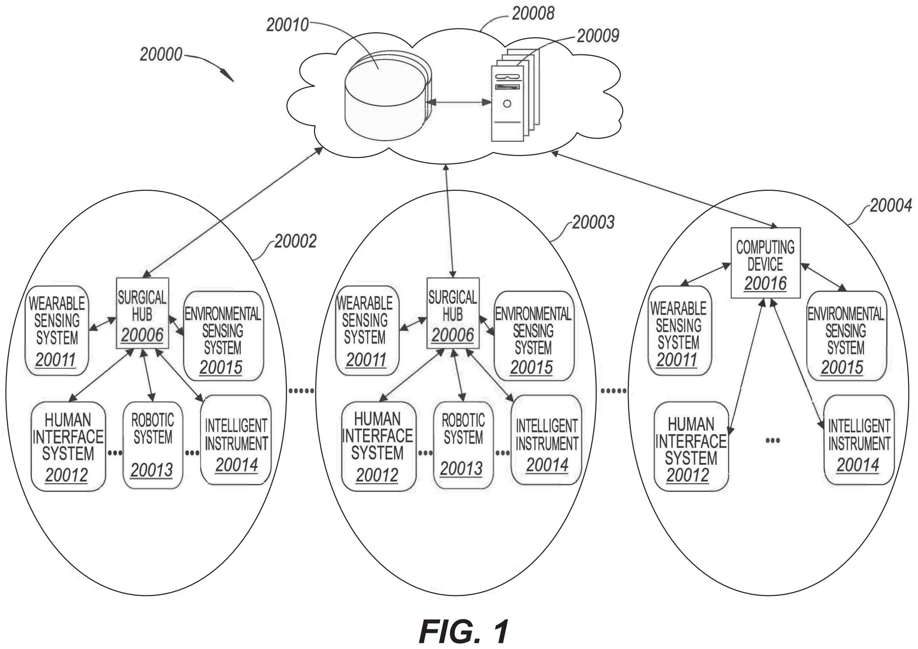

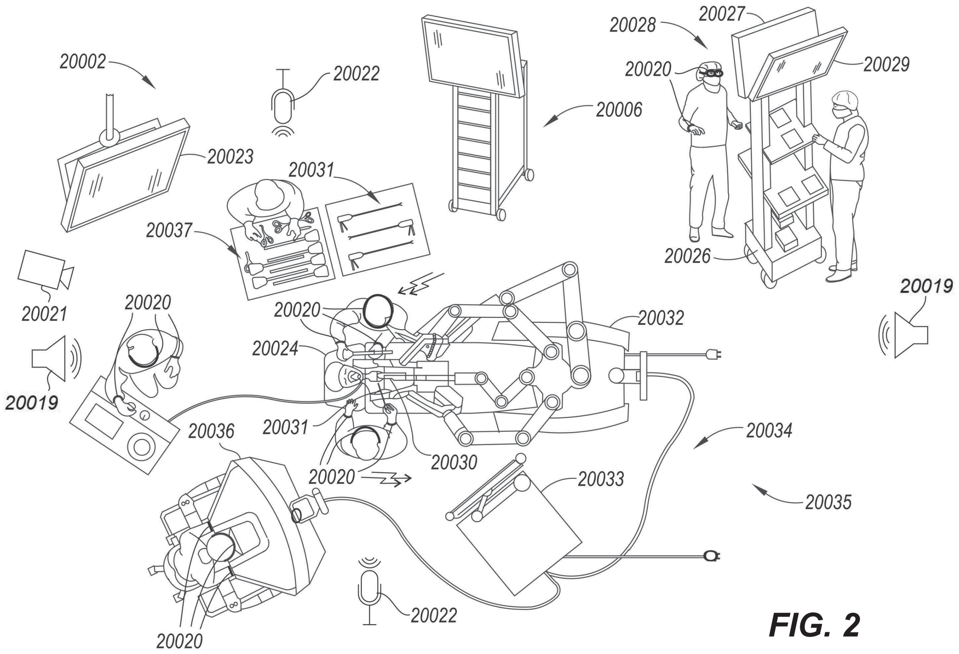

FIG. 1 shows an example computer-implemented surgical system 20000. The example surgical system 20000 may include one or more surgical systems (e.g., surgical sub-systems) 20002, 20003 and 20004. For example, surgical system 20002 may include a computer-implemented interactive surgical system. For example, surgical system 20002 may include a surgical hub 20006 and/or a computing device 20016 in communication with a cloud computing system 20008, for example, as described in FIG. 2. The cloud computing system 20008 may include at least one remote cloud server 20009 and at least one remote cloud storage unit 20010. Example surgical systems 20002, 20003, or 20004 may include one or more wearable sensing systems 20011, one or more environmental sensing systems 20015, one or more robotic systems 20013, one or more intelligent instruments 20014, one or more human interface systems 20012, etc. The human interface system is also referred herein as the human interface device. The wearable sensing system 20011 may include one or more health care professional (HCP) sensing systems, and/or one or more patient sensing systems. The environmental sensing system 20015 may include one or more devices, for example, used for measuring one or more environmental attributes, for example, as further described in FIG. 2. The robotic system 20013 may include a plurality of devices used for performing a surgical procedure, for example, as further described in FIG. 2.

The surgical system 20002 may be in communication with a remote server 20009 that may be part of a cloud computing system 20008. In an example, the surgical system 20002 may be in communication with a remote server 20009 via an internet service provider's cable/FIOS networking node. In an example, a patient sensing system may be in direct communication with a remote server 20009. The surgical system 20002 (and/or various sub-systems, smart surgical instruments, robots, sensing systems, and other computerized devices described herein) may collect data in real-time and transfer the data to cloud computers for data processing and manipulation. It will be appreciated that cloud computing may rely on sharing computing resources rather than having local servers or personal devices to handle software applications.

The surgical system 20002 and/or a component therein may communicate with the remote servers 20009 via a cellular transmission/reception point (TRP) or a base station using one or more of the following cellular protocols: GSM/GPRS/EDGE (2G), UMTS/HSPA (3G), long term evolution (LTE) or 4G, LTE-Advanced (LTE-A), new radio (NR) or 5G, and/or other wired or wireless communication protocols. Various examples of cloud-based analytics that are performed by the cloud computing system 20008, and are suitable for use with the present disclosure, are described in U.S. Patent Application Publication No. US 2019-0206569 A1 (U.S. Patent Application No. 16/209,403), titled METHOD OF CLOUD BASED DATA ANALYTICS FOR USE WITH THE HUB, filed December 4, 2018, the disclosure of which is herein incorporated by reference in its entirety.

The surgical hub 20006 may have cooperative interactions with one of more means of displaying the image from the laparoscopic scope and information from one or more other smart devices and one or more sensing systems 20011. The surgical hub 20006 may interact with one or more sensing systems 20011, one or more smart devices, and multiple displays. The surgical hub 20006 may be configured to gather measurement data from the sensing system(s) and send notifications or control messages to the one or more sensing systems 20011. The surgical hub 20006 may send and/or receive information including notification information to and/or from the human interface system 20012. The human interface system 20012 may include one or more human interface devices (HIDs). The surgical hub 20006 may send and/or receive notification information or control information to audio, display and/or control information to various devices that are in communication with the surgical hub.

For example, the sensing systems may include the wearable sensing system 20011 (which may include one or more HCP sensing systems and/or one or more patient sensing systems) and/or the environmental sensing system 20015 shown in FIG. 1. The sensing system(s) may measure data relating to various biomarkers. The sensing system(s) may measure the biomarkers using one or more sensors, for example, photosensors (e.g., photodiodes, photoresistors), mechanical sensors (e.g., motion sensors), acoustic sensors, electrical sensors, electrochemical sensors, thermoelectric sensors, infrared sensors, etc. The sensor(s) may measure the biomarkers as described herein using one of more of the following sensing technologies: photoplethysmography, electrocardiography, electroencephalography, colorimetry, impedimentary, potentiometry, amperometry, etc.

The biomarkers measured by the sensing systems may include, but are not limited to, sleep, core body temperature, maximal oxygen consumption, physical activity, alcohol consumption, respiration rate, oxygen saturation, blood pressure, blood sugar, heart rate variability, blood potential of hydrogen, hydration state, heart rate, skin conductance, peripheral temperature, tissue perfusion pressure, coughing and sneezing, gastrointestinal motility, gastrointestinal tract imaging, respiratory tract bacteria, edema, mental aspects, sweat, circulating tumor cells, autonomic tone, circadian rhythm, and/or menstrual cycle.

The biomarkers may relate to physiologic systems, which may include, but are not limited to, behavior and psychology, cardiovascular system, renal system, skin system, nervous system, gastrointestinal system, respiratory system, endocrine system, immune system, tumor, musculoskeletal system, and/or reproductive system. Information from the biomarkers may be determined and/or used by the computer-implemented patient and the surgical system 20000, for example. The information from the biomarkers may be determined and/or used by the computer-implemented patient and the surgical system 20000 to improve said systems and/or to improve patient outcomes, for example.

The sensing systems may send data to the surgical hub 20006. The sensing systems may use one or more of the following RF protocols for communicating with the surgical hub 20006: Bluetooth, Bluetooth Low-Energy (BLE), Bluetooth Smart, Zigbee, Z-wave, IPv6 Low-power wireless Personal Area Network (6LoWPAN), Wi-Fi.

The sensing systems, biomarkers, and physiological systems are described in more detail in U.S. App No. 17/156,287 (attorney docket number END9290USNP1), titled METHOD OF ADJUSTING A SURGICAL PARAMETER BASED ON BIOMARKER MEASUREMENTS, filed January 22, 2021, the disclosure of which is herein incorporated by reference in its entirety.

The sensing systems described herein may be employed to assess physiological conditions of a surgeon operating on a patient or a patient being prepared for a surgical procedure or a patient recovering after a surgical procedure. The cloud-based computing system 20008 may be used to monitor biomarkers associated with a surgeon or a patient in real-time and to generate surgical plans based at least on measurement data gathered prior to a surgical procedure, provide control signals to the surgical instruments during a surgical procedure, and notify a patient of a complication during post-surgical period.

The cloud-based computing system 20008 may be used to analyze surgical data. Surgical data may be obtained via one or more intelligent instrument(s) 20014, wearable sensing system(s) 20011, environmental sensing system(s) 20015, robotic system(s) 20013 and/or the like in the surgical system 20002. Surgical data may include, tissue states to assess leaks or perfusion of sealed tissue after a tissue sealing and cutting procedure pathology data, including images of samples of body tissue, anatomical structures of the body using a variety of sensors integrated with imaging devices and techniques such as overlaying images captured by multiple imaging devices, image data, and/or the like. The surgical data may be analyzed to improve surgical procedure outcomes by determining if further treatment, such as the application of endoscopic intervention, emerging technologies, a targeted radiation, targeted intervention, and precise robotics to tissue-specific sites and conditions. Such data analysis may employ outcome analytics processing and using standardized approaches may provide beneficial feedback to either confirm surgical treatments and the behavior of the surgeon or suggest modifications to surgical treatments and the behavior of the surgeon.

FIG. 2 shows an example surgical system 20002 in a surgical operating room. As illustrated in FIG. 2, a patient is being operated on by one or more health care professionals (HCPs). The HCPs are being monitored by one or more HCP sensing systems 20020 worn by the HCPs. The HCPs and the environment surrounding the HCPs may also be monitored by one or more environmental sensing systems including, for example, a set of cameras 20021, a set of microphones 20022, and other sensors that may be deployed in the operating room. The HCP sensing systems 20020 and the environmental sensing systems may be in communication with a surgical hub 20006, which in turn may be in communication with one or more cloud servers 20009 of the cloud computing system 20008, as shown in FIG. 1. The environmental sensing systems may be used for measuring one or more environmental attributes, for example, HCP position in the surgical theater, HCP movements, ambient noise in the surgical theater, temperature/humidity in the surgical theater, etc.

As illustrated in FIG. 2, a primary display 20023 and one or more audio output devices (e.g., speakers 20019) are positioned in the sterile field to be visible to an operator at the operating table 20024. In addition, a visualization/notification tower 20026 is positioned outside the sterile field. The visualization/notification tower 20026 may include a first non-sterile human interactive device (HID) 20027 and a second non-sterile HID 20029, which may face away from each other. The HID may be a display or a display with a touchscreen allowing a human to interface directly with the HID. A human interface system, guided by the surgical hub 20006, may be configured to utilize the HIDs 20027, 20029, and 20023 to coordinate information flow to operators inside and outside the sterile field. In an example, the surgical hub 20006 may cause an HID (e.g., the primary HID 20023) to display a notification and/or information about the patient and/or a surgical procedure step. In an example, the surgical hub 20006 may prompt for and/or receive input from personnel in the sterile field or in the non-sterile area. In an example, the surgical hub 20006 may cause an HID to display a snapshot of a surgical site, as recorded by an imaging device 20030, on a non-sterile HID 20027 or 20029, while maintaining a live feed of the surgical site on the primary HID 20023. The snapshot on the non-sterile display 20027 or 20029 can permit a non-sterile operator to perform a diagnostic step relevant to the surgical procedure, for example.

The surgical hub 20006 may be configured to route a diagnostic input or feedback entered by a non-sterile operator at the visualization tower 20026 to the primary display 20023 within the sterile field, where it can be viewed by a sterile operator at the operating table. In an example, the input can be in the form of a modification to the snapshot displayed on the non-sterile display 20027 or 20029, which can be routed to the primary display 20023 by the surgical hub 20006.

Referring to FIG. 2, a surgical instrument 20031 is being used in the surgical procedure as part of the surgical system 20002. The hub 20006 may be configured to coordinate information flow to a display of the surgical instrument(s) 20031. For example, in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. A diagnostic input or feedback entered by a non-sterile operator at the visualization tower 20026 can be routed by the hub 20006 to the surgical instrument display within the sterile field, where it can be viewed by the operator of the surgical instrument 20031. Example surgical instruments that are suitable for use with the surgical system 20002 are described under the heading "Surgical Instrument Hardware" and in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety, for example.

As shown in FIG. 2, the surgical system 20002 can be used to perform a surgical procedure on a patient who is lying down on an operating table 20024 in a surgical operating room 20035. A robotic system 20034 may be used in the surgical procedure as a part of the surgical system 20002. The robotic system 20034 may include a surgeon's console 20036, a patient side cart 20032 (surgical robot), and a surgical robotic hub 20033. The patient side cart 20032 can manipulate at least one removably coupled surgical tool 20037 through a minimally invasive incision in the body of the patient while the surgeon views the surgical site through the surgeon's console 20036. An image of the surgical site can be obtained by a medical imaging device 20030, which can be manipulated by the patient side cart 20032 to orient the imaging device 20030. The robotic hub 20033 can be used to process the images of the surgical site for subsequent display to the surgeon through the surgeon's console 20036.

Other types of robotic systems can be readily adapted for use with the surgical system 20002. Various examples of robotic systems and surgical tools that are suitable for use with the present disclosure are described herein, as well as in U.S. Patent Application Publication No. US 2019-0201137 A1 (U.S. Patent Application No. 16/209,407), titled METHOD OF ROBOTIC HUB COMMUNICATION, DETECTION, AND CONTROL, filed December 4, 2018, the disclosure of which is herein incorporated by reference in its entirety.

In various aspects, the imaging device 20030 may include at least one image sensor and one or more optical components. Suitable image sensors may include, but are not limited to, Charge-Coupled Device (CCD) sensors and Complementary Metal-Oxide Semiconductor (CMOS) sensors.

The optical components of the imaging device 20030 may include one or more illumination sources and/or one or more lenses. The one or more illumination sources may be directed to illuminate portions of the surgical field. The one or more image sensors may receive light reflected or refracted from the surgical field, including light reflected or refracted from tissue and/or surgical instruments.

The illumination source(s) may be configured to radiate electromagnetic energy in the visible spectrum as well as the invisible spectrum. The visible spectrum, sometimes referred to as the optical spectrum or luminous spectrum, is the portion of the electromagnetic spectrum that is visible to (e.g., can be detected by) the human eye and may be referred to as visible light or simply light. A typical human eye will respond to wavelengths in air that range from about 380 nm to about 750 nm.

The invisible spectrum (e.g., the non-luminous spectrum) is the portion of the electromagnetic spectrum that lies below and above the visible spectrum (i.e., wavelengths below about 380 nm and above about 750 nm). The invisible spectrum is not detectable by the human eye. Wavelengths greater than about 750 nm are longer than the red visible spectrum, and they become invisible infrared (IR), microwave, and radio electromagnetic radiation. Wavelengths less than about 380 nm are shorter than the violet spectrum, and they become invisible ultraviolet, x-ray, and gamma ray electromagnetic radiation.

In various aspects, the imaging device 20030 is configured for use in a minimally invasive procedure. Examples of imaging devices suitable for use with the present disclosure include, but are not limited to, an arthroscope, angioscope, bronchoscope, choledochoscope, colonoscope, cytoscope, duodenoscope, enteroscope, esophagogastro-duodenoscope (gastroscope), endoscope, laryngoscope, nasopharyngo-neproscope, sigmoidoscope, thoracoscope, and ureteroscope.

The imaging device may employ multi-spectrum monitoring to discriminate topography and underlying structures. A multi-spectral image is one that captures image data within specific wavelength ranges across the electromagnetic spectrum. The wavelengths may be separated by filters or by the use of instruments that are sensitive to particular wavelengths, including light from frequencies beyond the visible light range, e.g., IR and ultraviolet. Spectral imaging can allow extraction of additional information that the human eye fails to capture with its receptors for red, green, and blue. The use of multi-spectral imaging is described in greater detail under the heading "Advanced Imaging Acquisition Module" in U.S. Patent Application Publication No. US 2019-0200844 A1 (U.S. Patent Application No. 16/209,385), titled METHOD OF HUB COMMUNICATION, PROCESSING, STORAGE AND DISPLAY, filed December 4, 2018, the disclosure of which is herein incorporated by reference in its entirety. Multi-spectrum monitoring can be a useful tool in relocating a surgical field after a surgical task is completed to perform one or more of the previously described tests on the treated tissue. It is axiomatic that strict sterilization of the operating room and surgical equipment is required during any surgery. The strict hygiene and sterilization conditions required in a "surgical theater," e.g., an operating or treatment room, necessitate the highest possible sterility of all medical devices and equipment. Part of that sterilization process is the need to sterilize anything that comes in contact with the patient or penetrates the sterile field, including the imaging device 20030 and its attachments and components. It will be appreciated that the sterile field may be considered a specified area, such as within a tray or on a sterile towel, which is considered free of microorganisms, or the sterile field may be considered an area, immediately around a patient, who has been prepared for a surgical procedure. The sterile field may include the scrubbed team members, who are properly attired, and all furniture and fixtures in the area.

Wearable sensing system 20011 illustrated in FIG. 1 may include one or more HCP sensing systems 20020 as shown in FIG. 2. The HCP sensing systems 20020 may include sensing systems to monitor and detect a set of physical states and/or a set of physiological states of a healthcare personnel (HCP). An HCP may be a surgeon or one or more healthcare personnel assisting the surgeon or other healthcare service providers in general. In an example, an HCP sensing system 20020 may measure a set of biomarkers to monitor the heart rate of an HCP. In an example, an HCP sensing system 20020 worn on a surgeon's wrist (e.g., a watch or a wristband) may use an accelerometer to detect hand motion and/or shakes and determine the magnitude and frequency of tremors. The sensing system 20020 may send the measurement data associated with the set of biomarkers and the data associated with a physical state of the surgeon to the surgical hub 20006 for further processing.

The environmental sensing system(s) 20015 shown in FIG. 1 may send environmental information to the surgical hub 20006. For example, the environmental sensing system(s) 20015 may include a camera 20021 for detecting hand/body position of an HCP. The environmental sensing system(s) 20015 may include microphones 20022 for measuring the ambient noise in the surgical theater. Other environmental sensing system(s) 20015 may include devices, for example, a thermometer to measure temperature and a hygrometer to measure humidity of the surroundings in the surgical theater, etc. The surgeon biomarkers may include one or more of the following: stress, heart rate, etc. The environmental measurements from the surgical theater may include ambient noise level associated with the surgeon or the patient, surgeon and/or staff movements, surgeon and/or staff attention level, etc. The surgical hub 20006, alone or in communication with the cloud computing system, may use the surgeon biomarker measurement data and/or environmental sensing information to modify the control algorithms of hand-held instruments or the averaging delay of a robotic interface, for example, to minimize tremors.

The surgical hub 20006 may use the surgeon biomarker measurement data associated with an HCP to adaptively control one or more surgical instruments 20031. For example, the surgical hub 20006 may send a control program to a surgical instrument 20031 to control its actuators to limit or compensate for fatigue and use of fine motor skills. The surgical hub 20006 may send the control program based on situational awareness and/or the context on importance or criticality of a task. The control program may instruct the instrument to alter operation to provide more control when control is needed.

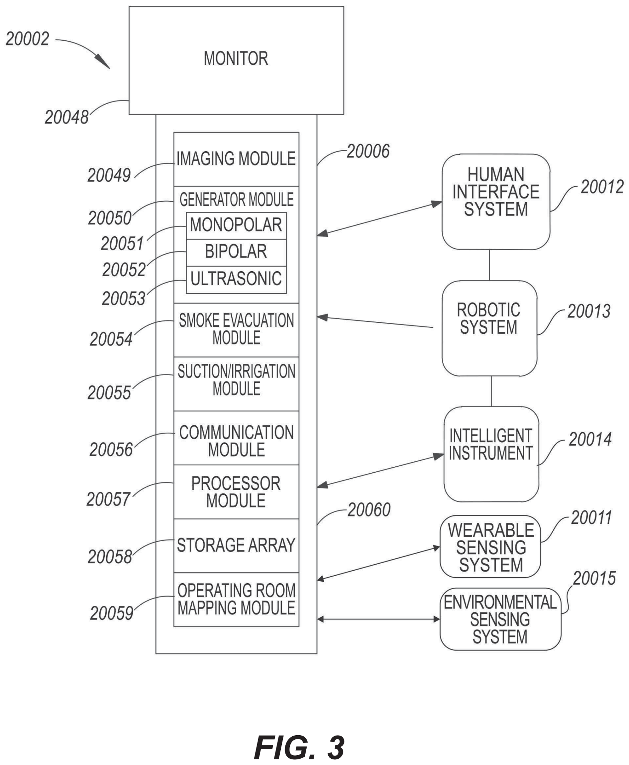

FIG. 3 shows an example surgical system 20002 with a surgical hub 20006. The surgical hub 20006 may be paired with, via a modular control, a wearable sensing system 20011, an environmental sensing system 20015, a human interface system 20012, a robotic system 20013, and an intelligent instrument 20014. The hub 20006 includes a display 20048, an imaging module 20049, a generator module 20050 (e.g., an energy generator), a communication module 20056, a processor module 20057, a storage array 20058, and an operating-room mapping module 20059. In certain aspects, as illustrated in FIG. 3, the hub 20006 further includes a smoke evacuation module 20054 and/or a suction/irrigation module 20055. The various modules and systems may be connected to the modular control either directly via a router or via the communication module 20056. The operating theater devices may be coupled to cloud computing resources and data storage via the modular control. The human interface system 20012 may include a display sub-system and a notification sub-system.

The modular control may be coupled to non-contact sensor module. The non-contact sensor module may measure the dimensions of the operating theater and generate a map of the surgical theater using ultrasonic, laser-type, and/or the like, non-contact measurement devices. Other distance sensors can be employed to determine the bounds of an operating room. An ultrasound-based non-contact sensor module may scan the operating theater by transmitting a burst of ultrasound and receiving the echo when it bounces off the perimeter walls of an operating theater as described under the heading "Surgical Hub Spatial Awareness Within an Operating Room" in U.S. Provisional Patent Application Serial No. 62/611,341, titled INTERACTIVE SURGICAL PLATFORM, filed December 28, 2017, which is herein incorporated by reference in its entirety. The sensor module may be configured to determine the size of the operating theater and to adjust Bluetooth-pairing distance limits. A laser-based non-contact sensor module may scan the operating theater by transmitting laser light pulses, receiving laser light pulses that bounce off the perimeter walls of the operating theater, and comparing the phase of the transmitted pulse to the received pulse to determine the size of the operating theater and to adjust Bluetooth pairing distance limits, for example.

During a surgical procedure, energy application to tissue, for sealing and/or cutting, may be associated with smoke evacuation, suction of excess fluid, and/or irrigation of the tissue. Fluid, power, and/or data lines from different sources may be entangled during the surgical procedure. Valuable time can be lost addressing this issue during a surgical procedure. Detangling the lines may necessitate disconnecting the lines from their respective modules, which may require resetting the modules. The hub modular enclosure 20060 may offer a unified environment for managing the power, data, and fluid lines, which reduces the frequency of entanglement between such lines.

Energy may be applied to tissue at a surgical site. The surgical hub 20006 may include a hub enclosure 20060 and a combo generator module slidably receivable in a docking station of the hub enclosure 20060. The docking station may include data and power contacts. The combo generator module may include two or more of: an ultrasonic energy generator component, a bipolar RF energy generator component, or a monopolar RF energy generator component that are housed in a single unit. The combo generator module may include a smoke evacuation component, at least one energy delivery cable for connecting the combo generator module to a surgical instrument, at least one smoke evacuation component configured to evacuate smoke, fluid, and/or particulates generated by the application of therapeutic energy to the tissue, and a fluid line extending from the remote surgical site to the smoke evacuation component. The fluid line may be a first fluid line, and a second fluid line may extend from the remote surgical site to a suction and irrigation module 20055 slidably received in the hub enclosure 20060. The hub enclosure 20060 may include a fluid interface.

The combo generator module may generate multiple energy types for application to the tissue. One energy type may be more beneficial for cutting the tissue, while another different energy type may be more beneficial for sealing the tissue. For example, a bipolar generator can be used to seal the tissue while an ultrasonic generator can be used to cut the sealed tissue. Aspects of the present disclosure present a solution where a hub modular enclosure 20060 is configured to accommodate different generators and facilitate an interactive communication therebetween. The hub modular enclosure 20060 may enable the quick removal and/or replacement of various modules.

The modular surgical enclosure may include a first energy-generator module, configured to generate a first energy for application to the tissue, and a first docking station comprising a first docking port that includes first data and power contacts, wherein the first energy-generator module is slidably movable into an electrical engagement with the power and data contacts and wherein the first energy-generator module is slidably movable out of the electrical engagement with the first power and data contacts. The modular surgical enclosure may include a second energy-generator module configured to generate a second energy, different than the first energy, for application to the tissue, and a second docking station comprising a second docking port that includes second data and power contacts, wherein the second energy generator module is slidably movable into an electrical engagement with the power and data contacts, and wherein the second energy-generator module is slidably movable out of the electrical engagement with the second power and data contacts. In addition, the modular surgical enclosure also includes a communication bus between the first docking port and the second docking port, configured to facilitate communication between the first energy-generator module and the second energy-generator module.

Referring to FIG. 3, the hub modular enclosure 20060 may allow the modular integration of a generator module 20050, a smoke evacuation module 20054, and a suction/irrigation module 20055. The hub modular enclosure 20060 may facilitate interactive communication between the modules 20059, 20054, and 20055. The generator module 20050 can be with integrated monopolar, bipolar, and ultrasonic components supported in a single housing unit slidably insertable into the hub modular enclosure 20060. The generator module 20050 may connect to a monopolar device 20051, a bipolar device 20052, and an ultrasonic device 20053. The generator module 20050 may include a series of monopolar, bipolar, and/or ultrasonic generator modules that interact through the hub modular enclosure 20060. The hub modular enclosure 20060 may facilitate the insertion of multiple generators and interactive communication between the generators docked into the hub modular enclosure 20060 so that the generators would act as a single generator.

A surgical data network having a set of communication hubs may connect the sensing system(s), the modular devices located in one or more operating theaters of a healthcare facility, a patient recovery room, or a room in a healthcare facility specially equipped for surgical operations, to the cloud computing system 20008.

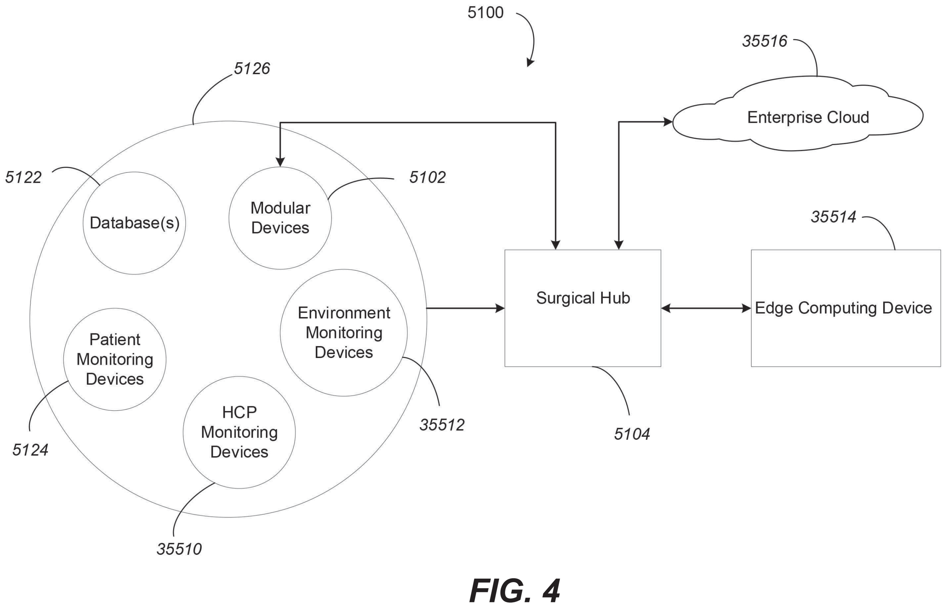

FIG. 4 illustrates a diagram of a situationally aware surgical system 5100. The data sources 5126 may include, for example, the modular devices 5102, databases 5122 (e.g., an EMR database containing patient records), patient monitoring devices 5124 (e.g., a blood pressure (BP) monitor and an electrocardiography (EKG) monitor), HCP monitoring devices 35510, and/or environment monitoring devices 35512. The modular devices 5102 may include sensors configured to detect parameters associated with the patient, HCPs and environment and/or the modular device itself. The modular devices 5102 may include one or more intelligent instrument(s) 20014. The surgical hub 5104 may derive the contextual information pertaining to the surgical procedure from the data based upon, for example, the particular combination(s) of received data or the particular order in which the data is received from the data sources 5126. The contextual information inferred from the received data can include, for example, the type of surgical procedure being performed, the particular step of the surgical procedure that the surgeon is performing, the type of tissue being operated on, or the body cavity that is the subject of the procedure. This ability by some aspects of the surgical hub 5104 to derive or infer information related to the surgical procedure from received data can be referred to as "situational awareness." For example, the surgical hub 5104 can incorporate a situational awareness system, which may be the hardware and/or programming associated with the surgical hub 5104 that derives contextual information pertaining to the surgical procedure from the received data and/or a surgical plan information received from the edge computing system 35514 or an enterprise cloud server 35516. The contextual information derived from the data sources 5126 may include, for example, what step of the surgical procedure is being performed, whether and how a particular modular device 5102 is being used, and the patient's condition.

The surgical hub 5104 may be connected to various databases 5122 to retrieve therefrom data regarding the surgical procedure that is being performed or is to be performed. In one exemplification of the surgical system 5100, the databases 5122 may include an EMR database of a hospital. The data that may be received by the situational awareness system of the surgical hub 5104 from the databases 5122 may include, for example, start (or setup) time or operational information regarding the procedure (e.g., a segmentectomy in the upper right portion of the thoracic cavity). The surgical hub 5104 may derive contextual information regarding the surgical procedure from this data alone or from the combination of this data and data from other data sources 5126.

The surgical hub 5104 may be connected to (e.g., paired with) a variety of patient monitoring devices 5124. In an example of the surgical system 5100, the patient monitoring devices 5124 that can be paired with the surgical hub 5104 may include a pulse oximeter (SpO2 monitor) 5114, a BP monitor 5116, and an EKG monitor 5120. The perioperative data that is received by the situational awareness system of the surgical hub 5104 from the patient monitoring devices 5124 may include, for example, the patient's oxygen saturation, blood pressure, heart rate, and other physiological parameters. The contextual information that may be derived by the surgical hub 5104 from the perioperative data transmitted by the patient moni-toring devices 5124 may include, for example, whether the patient is located in the operating theater or under anesthesia. The surgical hub 5104 may derive these inferences from data from the patient monitoring devices 5124 alone or in combination with data from other data sources 5126 (e.g., the ventilator 5118).

The surgical hub 5104 may be connected to (e.g., paired with) a variety of modular devices 5102. In one exemplification of the surgical system 5100, the modular devices 5102 that are paired with the surgical hub 5104 may include a smoke evacuator, a medical imaging device such as the imaging device 20030 shown in FIG. 2, an insufflator, a combined energy generator (for powering an ultrasonic surgical instrument and/or an RF electrosurgical instrument), and a ventilator.

The perioperative data received by the surgical hub 5104 from the medical imaging device may include, for example, whether the medical imaging device is activated and a video or image feed. The contextual information that is derived by the surgical hub 5104 from the perioperative data sent by the medical imaging device may include, for example, whether the procedure is a VATS procedure (based on whether the medical imaging device is activated or paired to the surgical hub 5104 at the beginning or during the course of the procedure). The image or video data from the medical imaging device (or the data stream representing the video for a digital medical imaging device) may be processed by a pattern recognition system or a machine learning system to recognize features (e.g., organs or tissue types) in the field of view (FOY) of the medical imaging device, for example. The contextual information that is derived by the surgical hub 5104 from the recognized features may include, for example, what type of surgical procedure (or step thereof) is being performed, what organ is being operated on, or what body cavity is being operated in.

The situational awareness system of the surgical hub 5104 may derive the contextual information from the data received from the data sources 5126 in a variety of different ways. For example, the situational awareness system can include a pattern recognition system, or machine learning system (e.g., an artificial neural network), that has been trained on training data to correlate various inputs (e.g., data from database(s) 5122, patient monitoring devices 5124, modular devices 5102, HCP monitoring devices 35510, and/or environment monitoring devices 35512) to corresponding contextual information regarding a surgical procedure. For example, a machine learning system may accurately derive contextual information regarding a surgical procedure from the provided inputs. In examples, the situational awareness system can include a lookup table storing pre-characterized contextual information regarding a surgical procedure in association with one or more inputs (or ranges of inputs) corresponding to the contextual information. In response to a query with one or more inputs, the lookup table can return the corresponding contextual information for the situational awareness system for controlling the modular devices 5102. In examples, the contextual information received by the situational awareness system of the surgical hub 5104 can be associated with a particular control adjustment or set of control adjustments for one or more modular devices 5102. In examples, the situational awareness system can include a machine learning system, lookup table, or other such system, which may generate or retrieve one or more control adjustments for one or more modular devices 5102 when provided the contextual information as input.

For example, based on the data sources 5126, the situationally aware surgical hub 5104 may determine what type of tissue was being operated on. The situationally aware surgical hub 5104 can infer whether a surgical procedure being performed is a thoracic or an abdominal procedure, allowing the surgical hub 5104 to determine whether the tissue clamped by an end effector of the surgical stapling and cutting instrument is lung (for a thoracic procedure) or stomach (for an abdominal procedure) tissue. The situationally aware surgical hub 5104 may determine whether the surgical site is under pressure (by determining that the surgical procedure is utilizing insufflation) and determine the procedure type, for a consistent amount of smoke evacuation for both thoracic and abdominal procedures. Based on the data sources 5126, the situationally aware surgical hub 5104 could determine what step of the surgical procedure is being performed or will subsequently be performed.

The situationally aware surgical hub 5104 could determine what type of surgical procedure is being performed and customize the energy level according to the expected tissue profile for the surgical procedure. The situationally aware surgical hub 5104 may adjust the energy level for the ultrasonic surgical instrument or RF electrosurgical instrument throughout the course of a surgical procedure, rather than just on a procedure-by-procedure basis.

In examples, data can be drawn from additional data sources 5126 to improve the conclusions that the surgical hub 5104 draws from one data source 5126. The situationally aware surgical hub 5104 could augment data that it receives from the modular devices 5102 with contextual information that it has built up regarding the surgical procedure from other data sources 5126.

The situational awareness system of the surgical hub 5104 can consider the physiological measurement data to provide additional context in analyzing the visualization data. The additional context can be useful when the visualization data may be inconclusive or incomplete on its own.

The situationally aware surgical hub 5104 could determine whether the surgeon (or other HCP(s)) was making an error or otherwise deviating from the expected course of action during the course of a surgical procedure. For example, the surgical hub 5104 may determine the type of surgical procedure being performed, retrieve the corresponding list of steps or order of equipment usage (e.g., from a memory), and compare the steps being performed or the equipment being used during the course of the surgical procedure to the expected steps or equipment for the type of surgical procedure that the surgical hub 5104 determined is being performed. The surgical hub 5104 can provide an alert indicating that an unexpected action is being performed or an unexpected device is being utilized at the particular step in the surgical procedure.

The surgical instruments (and other modular devices 5102) may be adjusted for the particular context of each surgical procedure (such as adjusting to different tissue types) and validating actions during a surgical procedure. Next steps, data, and display adjustments may be provided to surgical instruments (and other modular devices 5102) in the surgical theater according to the specific context of the procedure.

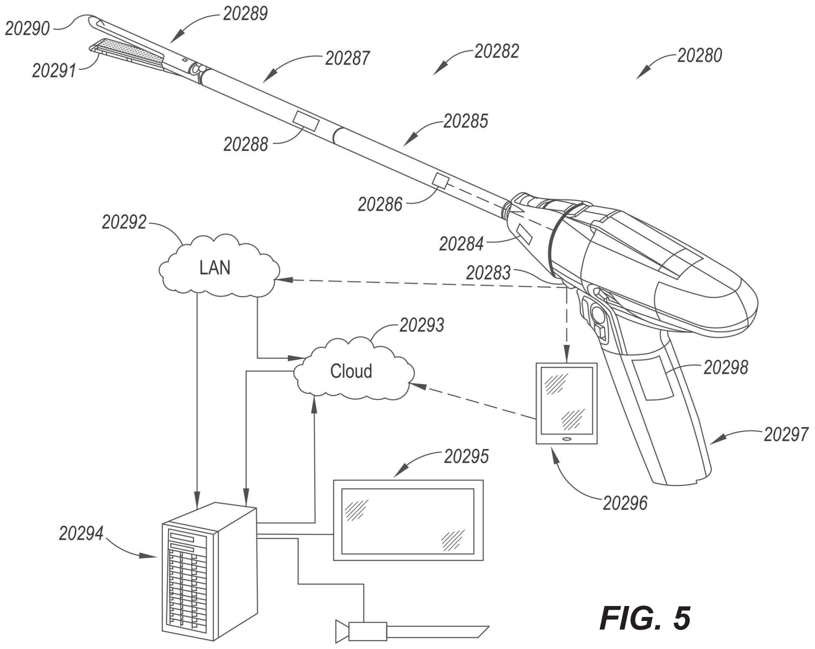

FIG. 5 illustrates an example surgical system 20280 that may include a surgical instrument 20282. The surgical instrument 20282 can be in communication with a console 20294 and/or a portable device 20296 through a local area network 20292 and/or a cloud network 20293 via a wired and/or wireless connection. The console 20294 and the portable device 20296 may be any suitable computing device. Surgical instrument 20282 may include a handle 20297, an adapter 20285, and a loading unit 20287. The adapter 20285 releasably couples to the handle 20297 and the loading unit 20287 releasably couples to the adapter 20285 such that the adapter 20285 transmits a force from a drive shaft to the loading unit 20287. The adapter 20285 or the loading unit 20287 may include a force gauge (not explicitly shown) disposed therein to measure a force exerted on the loading unit 20287. The loading unit 20287 may include an end effector 20289 having a first jaw 20291 and a second jaw 20290. The loading unit 20287 may be an in-situ loaded or multi-firing loading unit (MFLU) that allows a clinician to fire a plurality of fasteners multiple times without requiring the loading unit 20287 to be removed from a surgical site to reload the loading unit 20287.

The first and second jaws 20291, 20290 may be configured to clamp tissue therebetween, fire fasteners through the clamped tissue, and sever the clamped tissue. The first jaw 20291 may be configured to fire at least one fastener a plurality of times or may be configured to include a replaceable multi-fire fastener cartridge including a plurality of fasteners (e.g., staples, clips, etc.) that may be fired more than one time prior to being replaced. The second jaw 20290 may include an anvil that deforms or otherwise secures the fasteners, as the fasteners are ejected from the multi-fire fastener cartridge.

The handle 20297 may include a motor that is coupled to the drive shaft to affect rotation of the drive shaft. The handle 20297 may include a control interface to selectively activate the motor. The control interface may include buttons, switches, levers, sliders, touchscreens, and any other suitable input mechanisms or user interfaces, which can be engaged by a clinician to activate the motor.

The control interface of the handle 20297 may be in communication with a controller 20298 of the handle 20297 to selectively activate the motor to affect rotation of the drive shafts. The controller 20298 may be disposed within the handle 20297 and may be configured to receive input from the control interface and adapter data from the adapter 20285 or loading unit data from the loading unit 20287. The controller 20298 may analyze the input from the control interface and the data received from the adapter 20285 and/or loading unit 20287 to selectively activate the motor. The handle 20297 may also include a display that is viewable by a clinician during use of the handle 20297. The display may be configured to display portions of the adapter or loading unit data before, during, or after firing of the instrument 20282.

The adapter 20285 may include an adapter identification device 20284 disposed therein and the loading unit 20287 may include a loading unit identification device 20288 disposed therein. The adapter identification device 20284 may be in communication with the controller 20298, and the loading unit identification device 20288 may be in communication with the controller 20298. It will be appreciated that the loading unit identification device 20288 may be in communication with the adapter identification device 20284, which relays or passes communication from the loading unit identification device 20288 to the controller 20298.

The adapter 20285 may also include a plurality of sensors 20286 (one shown) disposed thereabout to detect various conditions of the adapter 20285 or of the environment (e.g., if the adapter 20285 is connected to a loading unit, if the adapter 20285 is connected to a handle, if the drive shafts are rotating, the torque of the drive shafts, the strain of the drive shafts, the temperature within the adapter 20285, a number of firings of the adapter 20285, a peak force of the adapter 20285 during firing, a total amount of force applied to the adapter 20285, a peak retraction force of the adapter 20285, a number of pauses of the adapter 20285 during firing, etc.). The plurality of sensors 20286 may provide an input to the adapter identification device 20284 in the form of data signals. The data signals of the plurality of sensors 20286 may be stored within or be used to update the adapter data stored within the adapter identification device 20284. The data signals of the plurality of sensors 20286 may be analog or digital. The plurality of sensors 20286 may include a force gauge to measure a force exerted on the loading unit 20287 during firing.

The handle 20297 and the adapter 20285 can be configured to interconnect the adapter identification device 20284 and the loading unit identification device 20288 with the controller 20298 via an electrical interface. The electrical interface may be a direct electrical interface (i.e., include electrical contacts that engage one another to transmit energy and signals therebetween). Additionally, or alternatively, the electrical interface may be a non-contact electrical interface to wirelessly transmit energy and signals therebetween (e.g., inductively transfer). It is also contemplated that the adapter identification device 20284 and the controller 20298 may be in wireless communication with one another via a wireless connection separate from the electrical interface.

The handle 20297 may include a transceiver 20283 that is configured to transmit instrument data from the controller 20298 to other components of the system 20280 (e.g., the LAN 20292, the cloud 20293, the console 20294, or the portable device 20296). The controller 20298 may also transmit instrument data and/or measurement data associated with one or more sensors 20286 to a surgical hub. The transceiver 20283 may receive data (e.g., cartridge data, loading unit data, adapter data, or other notifications) from the surgical hub 20270. The transceiver 20283 may receive data (e.g., cartridge data, loading unit data, or adapter data) from the other components of the system 20280. For example, the controller 20298 may transmit instrument data including a serial number of an attached adapter (e.g., adapter 20285) attached to the handle 20297, a serial number of a loading unit (e.g., loading unit 20287) attached to the adapter 20285, and a serial number of a multi-fire fastener cartridge loaded into the loading unit to the console 20294. Thereafter, the console 20294 may transmit data (e.g., cartridge data, loading unit data, or adapter data) associated with the attached cartridge, loading unit, and adapter, respectively, back to the controller 20298. The controller 20298 can display messages on the local instrument display or transmit the message, via transceiver 20283, to the console 20294 or the portable device 20296 to display the message on the display 20295 or portable device screen, respectively.

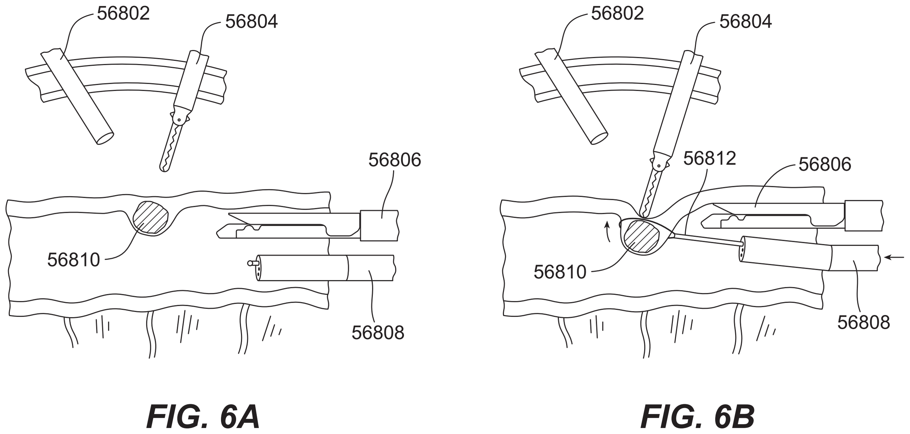

FIG. 6A illustrates an example surgical step for a tumor resection procedure. Tumor 56810 may be located within a patient's tissue and surrounded by various surgical instruments introduced through corresponding access points. Endoscope 56806 may be positioned proximal to tumor 56810 and facilitate visualization and access to the surgical site. A first trocar 56802 may accommodate the insertion of a laparoscope, which may assist in navigation and provide a direct view of the tumor and/or surrounding anatomy. A second trocar 56804 may accommodate a laparoscopic grasper configured for manipulating tissue and holding tumor 56810. Stapler 56808 may be inserted through a third trocar or an auxiliary access point to facilitate transection or securing of tissue proximal to tumor 56810. The system may enable precise instrument positioning and movement during the procedure. The example setup shown in FIG. 6A may illustrate an approach where instruments are positioned strategically to allow for control (e.g., maximum control) and access for subsequent steps in the surgical procedure.

FIG. 6B illustrates an example surgical step for a tumor resection procedure (e.g., an intermediate stage in the procedure). Tumor 56810 may be manipulated and inverted using the laparoscopic grasper inserted through trocar 56804. The inversion may expose the tumor for excision while providing clear visualization and access. A Snare 56812 may encircle and excise tumor 56810. Stapler 56808 may secure surrounding tissue to prevent unnecessary movement or bleeding during the excision. The system may incorporate robotic assistance to coordinate movements of the laparoscope, grasper, snare, and stapler for their respective actions. The positional configurations of the instruments may be adjusted based on feedback from the endoscope and other sensors to enable alignment and access during the procedure. Patient-specific anatomic variations, instrument articulation limits, and trocar placements may influence the overall surgical approach.

The surgical setup depicted in FIGS. 6A and 6B may correspond to how positional and orientation configurations may be determined and adjusted during the procedure, including the placement of trocars 56802, 56804, and auxiliary access points/trocar placement for stapler 56808, for example. For example, the system may identify tumor resection involving tumor 56810 as the active surgical step and determine positional configurations for a laparoscopic grasper inserted through trocar 56804, endoscope 56806, and stapler 56808. The placement of trocars 56802 and 56804 may be calculated based on the orientation of tumor 56810, patient-specific anatomy, and the parameters of downstream surgical steps, such as applying snare 56812 or securing tissue. For example, the insertion of laparoscopic grasper through trocar 56804 may correspond to a selected configuration that allows manipulation and inversion of tumor 56810. The placement of stapler 56808 relative to the patient and tumor 56810 may enable access and alignment for securing tissue during or after resection. These placements and adjustments may be informed by feedback from endoscope 56806 and other sensors to refine positional and orientation configurations. The system may display the anticipated effects of these configurations, including trocar placement on tumor 56810 and surrounding anatomy, allowing the user to make an informed decision and execute control over the surgical procedure. Generally, downstream positional configurations (and/or orientation configurations) can be determined based on an articulation range and/or one or more reach limitations of the surgical instrument. Generally, downstream positional configurations (and/or orientation configurations) can be determined by calculating the configurations that are reachable from each of the positional configurations (and/or orientation configurations) associated with the active surgical step, or by calculating the configurations that are reachable from each of the positional configurations (and/or orientation configurations) associated with the active surgical step without moving or re-placing a trocar or other port. Generally, downstream positional configurations (and/or orientation configurations) can be determined based on the configurations capable of performing, or optimal for performing, the next surgical step. Generally, downstream positional configurations (and/or orientation configurations) can be determined based on one or more known limitations on the surgical instrument's degrees of freedom.

Smart systems (e.g., a first smart system and a second smart system) may work together, through machine learning and surgeon profile, to allow robotic arms to move to a (e.g., desired or learned) location from a surgery (e.g., a previous similar or same surgery). Robotic arms may automatically position at the beginning of the case based on visual feedback from an above arm-mounted camera, such as a laparoscope mounted on the center arm. Zeroing on a site (e.g., an umbilicus), the system may position the remaining arms to move into position based on a learned procedure and surgeon profile. The arms may include a laser light to project onto the patient abdomen to a location (e.g., a best location with respect to the patient or the procedure) for the trocar placement.

In examples, a system may include a module that stores procedure data, robotically controlled arms for laparoscopic instrumentation, a camera (e.g., within the arms or arm containing multiple arms), a light source on an arm to project an emitting laser light or beam, and an algorithm that may position the arms based on the data from a previous learned procedure data.

The display may present a visual representation of the surgical site, including a real-time depiction of the instruments and their positions relative to a surgical subject, such as, for example, tumor 56810, and surrounding anatomy (e.g., the display may depict the setup shown in FIG. 6B while 6A is being actively conducted for aiding the user about a subsequent step in the surgical procedure). For example, the display may include a dynamic overlay indicating the positional configurations of endoscope 56806, laparoscopic grasper through trocar 56804, stapler 56808, and snare 56812. The overlay may highlight critical structures, such as tumor 56810, using visual cues like shading, borders, or labels to emphasize areas of interest. The display may include annotations or visual markers showing the placement of trocars 56802, 56804, and auxiliary access points, along with their respective angles and distances from the tumor, allowing the user to assess alignment and access options visually.

The display may include real-time feedback and predictive analytics to aid user decision-making. For example, the display may show anticipated effects of selected positional configurations on tumor 56810 and surrounding anatomy, such as the trajectory of the laparoscopic grasper during inversion or the predicted tissue compression from stapler 56808. Warnings or alerts may appear if a configuration is determined to be unviable, with visual indicators explaining the cause, such as articulation limits or improper trocar alignment. Alternative configurations may be displayed alongside, for example, using a side-by-side comparison or visualization, showing how adjustments in trocar placement or instrument orientation may impact access or surgical outcomes. The display may include a simulated or augmented view of the next surgical step, such as the application of snare 56812.

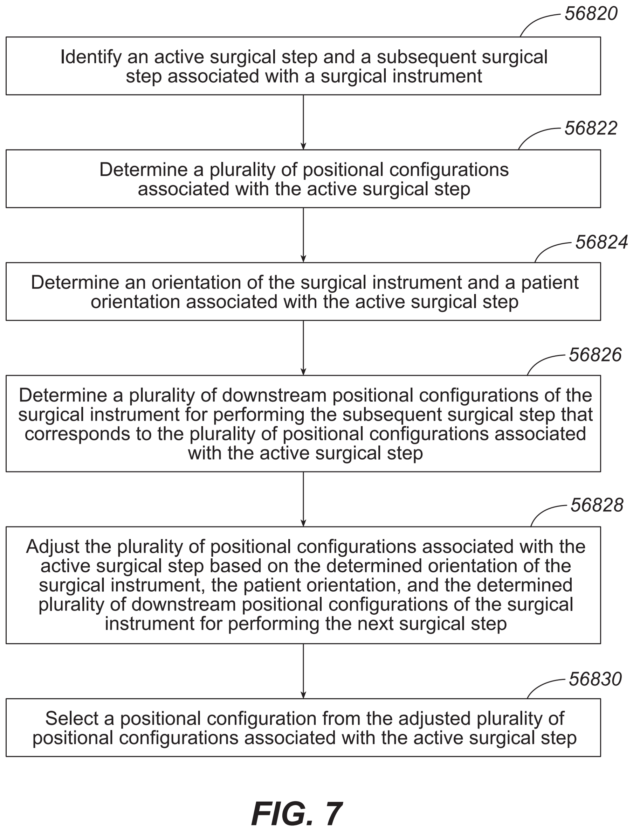

FIG. 7 shows an example flowchart associated with the operation of a system (e.g., referred to interchangeably herein as a smart system, surgical system, surgical smart system, smart surgical system). At 56820, the system may identify an active surgical step and a subsequent surgical step associated with a surgical instrument. At 56822, the system may determine positional configurations associated with the active surgical step. At 56824, the system may determine an orientation of the surgical instrument and a patient orientation associated with the active surgical step. At 56826, the system may determine downstream positional configurations of the surgical instrument for performing the subsequent surgical step that corresponds to the positional configurations associated with the active surgical step. At 56828, the system may adjust the positional configurations associated with the active surgical step based on the determined orientation of the surgical instrument, the patient orientation, and the determined downstream positional configurations of the surgical instrument for performing the next surgical step. At 56830, the system may select a positional configuration from the adjusted positional configurations associated with the active surgical step.

The system may determine anticipated effects based on the positional configurations associated with the active surgical step. The device may generate a display notification configured to display the anticipated effects on an anatomic shape. The device may receive an input based on the display notification, and the positional configuration may be selected based on the received input. The device may generate a control signal configured to maneuver the surgical instrument to move in accordance with the selected positional configuration.

Patient anatomical variability and surgeon technical errors may determine transection pathways and keep away areas. In examples, surgeons performing laparoscopic cases may be associated with speed of learning for resident training. An IMU on the device may provide a way for the IMU to display information on the monitor. Dropping a pin that highlights the path the surgeon is planning on taking on the display may be implemented.

Maintaining relative position to anatomy in laparoscopic and robotic procedures where orientation changes throughout the procedure may reduce inadvertent tissue injury and dissection in a tissue plane (e.g., a wrong tissue plane). An IMU on the device may display information on the monitor, and dropping a tag may auto-track the point or tissue type throughout the procedure.

Identifying persisting without progress may assist surgeons performing laparoscopic and robotic procedures for surgeon learning and procedure efficiency. An IMU on the device may be used with a surgeon portal for post-operative assessment delivery. Tracking expert surgeon movement as a baseline and post-operative assessment of how efficient the surgeon was during a surgical procedure may be implemented, including tracking returns to the same point in space.

Identifying users based on technique or mannerisms (e.g., resident, attending, tech, etc.) in a case with more than one operator may be used in feedback and residency competency acceleration. Utilizing movement information to differentiate users or user groups may be used, including attending feedback for resident performance during training.

Detecting user behaviors prior to critical steps or instrument use in cases with Ethicon laparoscopic devices (e.g., end cutters, energy devices) may reassure the user or surgeon, aid in clinical decision-making, and reduce moving away from the target structure during the case. Detecting patterns of use may be described herein. Recording surgeon movements, behaviors, and audio prior to critical moments, steps, and landscape assessments with devices may be considered. For example, a surgeon may move from the target structure to check a structure prior to performing a task.

Detecting motion that can cause blunt trauma in patients at risk of blunt trauma (e.g., bowel, liver injury from trocar, undetected injury) may reduce patient injury and blunt trauma injuries. Utilizing a Presto indicator for speed may be applied; for example, detecting when a port is moving too quickly may be associated with characterizing blunt trauma.

An indication or limitation of motions that would result in intolerable device-to-device or device-to-patient interactions may be provided. Access port restriction of instrument mobility may be considered. Surgical site reach limits based on patient position and access port positioning may be determined, including combination analysis of trocar and tool limitations for access calculations. If the system has knowledge of a trocar placement, as well as a tool correlated to such a trocars, the system may know the limits of access that can be reached from such locations. For example, if a McLaren 60 Long is inserted into a trocar located in the left abdomen, the system may know the length of the cartridge, as the shaft, and the limits of articulation of the end effector as well (e.g., 55 degrees based on McLaren). The system may understand, based on a placement (e.g., of the trocar), where the patient's anatomy may be accessed. Approach angle limits may be based on tilt ability of the trocar.

Examples described herein may be associated with percutaneous nephrolithotomy (PCNL) conversion and percutaneous transluminal approaches. Proactively modifying port placement may aid with port placement in laparoscopic cases performed by residents or early career surgeons. Port placement may be associated with case efficiency (e.g., time to change port placement), reducing patient injury (e.g., flank pain), and surgeon/resident confidence. A placement may be oriented and/or calculated based on moving the device outside of the body (ex vivo). An overlay may show a placement of trocars. During pre-op setup for the case, a body landmark (e.g., the umbilicus) may be used to determine trocar placement. Surgeons may use a motion sensor algorithm to detect or predict port placement for laparoscopic procedures.

The device may determine orientation configurations associated with the active surgical step. The device may determine downstream orientation options of the surgical instrument for performing the subsequent surgical step that correspond to the orientation options associated with the active surgical step. The device may adjust the orientation options associated with the active surgical step based on the determined orientation of the surgical instrument, the patient orientation, and the determined downstream orientation options of the surgical instrument for performing the next surgical step. The device may select an orientation option from the adjusted orientation options associated with the active surgical step.

Visualization source adaptive orientation control may be used to maintain a fix on the moving surgical field of operation. Keeping the target field of view in the center of focus may be implemented, for example, when using a robotically controlled camera (e.g., and may be associate with a (e.g., positive) surgeon experience, procedure efficiency, and (e.g., a reduction of) interruptions from fixing visualization). An IMU on the device, IMU, and camera, may be used to auto-track the device for cameras that are robotically controlled.

Features described herein may be associated with centering the intended surgical view within a monitor. Potential targets may include anatomy, such as stomach, bowel, organs, items for removal, devices, graspers, endocutters, energy tools, and fiducial marking systems. The surgeon may draw or insert an ink or biomarker to track anatomy, including collagen marking and ICG. Artificially created surgical areas, location between trocars, geofenced zones, and surgical staff-selected areas may also be considered.

Prioritization and establishment of the intended surgical view may be achieved through various methods. Surgical staff selection may involve the surgeon or staff providing input (e.g., such as on a console or tablet) to select a target focus. Surgeon selection of a tool to track may involve the surgeon drawing on the screen of a region or area within the center for highlighting and identifying as an area of interest. Tool-focused approaches may follow a tool such as a grasper, such that focus is maintained. Button control or source control of the selected tool may be utilized, along with artificial tool highlighting to show which tool is selected. Examples described herein may include highlighting of the console overlay versus highlighting the overlay of the tool in the image. Camera identification of devices within the view (e.g., versus a pre-existing list of tools being used within a surgery) may be described herein. Tools may be marked with a fiducial and, for example, a snap-to devices with errors may be marked by the fiducial.

Examples described herein may include the focus area being shifted as the system identifies procedural steps. The system may be zoomed in for a staple line firing and after the firing, the system zooms back out while the cartridge is being replaced. Multifactorial inputs may cause system focus to change depending on what is in view currently. If the anatomy is in view, the system may track anatomy and shift focus once an instrument is in view. Localized targeting based on zoom may involve the system targeting anatomy, and targeting may change based on zoom. In examples, specific targets may be overly focused at far zoom-out levels (e.g., showing an area of the stomach), and when zoomed in, a biomarker may be (e.g., specifically focused on).

For types of compensation, examples described herein may include compensatory effects for visual stabilization within a moving surgical field. Movement of areas may include detailed camera movement, including camera rotation as the camera is rotated around, camera pitch and yaw as the camera has pitch or yaw, and camera swivel as the camera swivels around the topic. Proximity, e.g., the distance of the camera from the intended target, may be relevant. Jitter or bounce may occur when the camera is being exposed to bounce or physical distortion. Video latency and split screen or multi-view may affect visual stabilization.

Target (e.g., anatomy) movement adjustment may include orientation change, fold over, location shift, and proximity (e.g., distance). Compensatory effects may include digital debouncing of the camera, where the camera system may display a portion of the image and reserve a portion of the image as a buffer to enable the final image presented to the user to be debounced. Camera movement speed control may be adjusted; as the camera shifts to adjust its view and track a target, the speed at which the camera does so may be adjustable to enable (e.g., fluid) display of the video feed. Maintaining views of anatomy that are moving without moving the camera may include reconstruction of images.

Camera orientation to match the orientation of a defined device may be described herein. The camera may continue to orient itself to match the change in orientation of a determined device, such as the top of an endocutter. Surgeon preferences within a certain range may be accommodated; the camera may track objects to the surgeon's preference. For example, the camera may constantly track an object, and a surgeon may prefer that the camera track at points (e.g., specific points).

Time-related versus distance-from-center-related adjustments may be considered. Regarding time, the camera readjusts at certain time intervals. In terms of distance from center, the camera readjusts as the target gets farther from the center of the camera orientation. Focal adjustments may address how the camera knows how the surgeon can clearly see the field. Auto-focus capabilities may include auto-focus of the general camera view and focus to the intended target. Center versus feature-based autofocus may be used; if a target has been identified, the zoom adjustments may be automatically adjusted to allow the targeted feature to maintain a certain percentage of the screen space. Lighting adjustments and spectrum adjustments may also be applied.

A priority may be modified, and a predefined prioritization of the camera view may be changed based on risk. Camera faults may result in the camera going to black screen. A higher risk event may be detected that is more important to focus on than the current focus. Surgeon preferences may influence prioritization.

If the intended target can no longer be tracked, error controls and other measures may be taken. Camera actions may include automatic switch to a secondary target or switch to manual mode. If the intended target cannot be tracked, the system may pause at the last known location. Escalation of actions may occur; the camera may no longer track movement within its field of view. In a robotic camera system, the arm may reposition, and an arm (e.g., other arms) may reposition anatomy. Alerts and guidance to the surgeon may be provided.

The device may determine anticipated effects based on the orientation options associated with the active surgical step. The device may generate a display notification configured to display the anticipated effects on an anatomic shape. The device may receive an input based on the display notification, and the orientation option may be selected based on the received input. The device may generate a control signal configured to maneuver the surgical instrument to move in accordance with the selected orientation option. The device may determine that a positional configuration of the positional configurations is unviable based on the active surgical step and the next surgical step. The device may identify a cause associated with the positional configuration being unviable, and the positional configurations may be adjusted based on the positional configuration being unviable and the cause associated with the positional configuration being unviable. The device may generate a display notification that displays the cause associated with the positional configuration being unviable. The cause may include one or more a reach, an articulation range, a port utilization, a port placement, an anatomic shape, an anatomic thickness, or a previous surgical step.

Measuring mesh may be associated with hernia surgical repairs (e.g., with respect to efficiency and using, for example, point-to-point measurement and hernia mesh sizing tool or assistance). Measuring pylorus to staple distance in lap sleeve gastrectomy (LSG) may reduce incidence of de novo gastroesophageal reflux disease (GERD). Point-to-point anatomic measurement and a lap sleeve gastrectomy may be considered.

Measuring bowel in Roux-en-Y procedures may determine measurement for patient weight loss, which may be associated with bowel remaining in patient. Point measurements and speed of device may be pulled in relation to length of tissue pulled. Examples described herein may be associated with confirming bowel length in Roux-en-Y procedures; resection may equate to dumping syndrome, and little dumping may not be associated with weight loss.

Measuring distances of critical structures or target tissue in laparoscopic cases may be associated with efficiency, (e.g., reducing) human performance deficiencies, and clinical decision-making. Utilizing movement to measure distances may be implemented. Surgeons may measure aspects such as whether a measurement is 4mm or 6mm, margins, and distances to structures (e.g., bowel).

Measuring mobility of critical structures or target tissue in laparoscopic cases may be associated with clinical outcomes, (e.g., reducing) human performance deficiencies, and clinical decision-making. Utilizing movement to measure mobility of tissue or critical structures may be considered. The tool may be used to measure the mobility of anatomical features; for example, dissection for perfusion results, tension (e.g., amount of tension), and reach.

Monitoring the orientation of a smart instrument relative to the surgeon or relative to the patient from a first smart system (e.g., which is exchanged with a second smart system being controlled by the surgeon), may utilize the data to adjust aspects of the control of function to compensate for the data received. Controls may be adapted due to awareness of data from the hub.

For example, an inverted endocutter may be viewed by the room camera along with the posture of the surgeon and which fingers are accessing the firing trigger to activate (e.g., pinky versus index). The operation may adjust its behavior based on such information. Because of the data from the room cameras, the endocutter may instruct that there is a reach issue, and the system may adjust how much the trigger is to be pulled in order to start or maintain activation. In an Echelon 3000, the trigger may be activated and maintain the amount of force or displacement on the trigger throughout firing, or the trigger may stop mid-fire.

For example, as the endocutter approaches an articulation limit, the endocutter may inform the visualization system. The visualization system may suggest articulating to a side and rotating the head 180 degrees to improve access rather than suggesting articulating (e.g., articulate further) when the user presses a button to articulate further in a direction (e.g., in an initial direction).

Monitoring of a smart device having user input controls and an understanding of its actuation location and limits may be associated with a hub with data on further motions and user-specified control instructions. The hub may change the proposed instructions for how to access the device beyond its physical limits. Changing the hub may include adaptations of hub displays due to awareness of smart instrument controls and actuation limits.

Examples described herein may be associated with providing laparoscopic measurements of anatomy or relative instrument measurements for tasks or jobs. Measuring angles may be associated with surgeons performing laparoscopic surgery for consistency of manipulation in angles associated with patient outcomes and surgeon longevity. An IMU on disposable instruments or a 2D camera in the room may be used. Performance metrics related to angles for triangulation and ergonomics may be analyzed. In examples, questions may be asked to the surgeon, for example, "Is table wrong height, could you rotate patient" and "Is it more stressful on you vs. your peers" may be addressed. Manipulation angle-between 90 and 45 degrees, for example-and angle with respect to gravity, may be measured.

Providing advice on device placement or surgical job direction and positioning may include determining transection pathways and keepaway areas. Surgeons performing lap cases may be associated with the speed (e.g., improving the speed) of learning for resident training. An IMU on the device may provide a means for the IMU to display information on the monitor, including dropping a pin that highlights the path the surgeon is planning on taking on the display.

Defining transection pathways in bariatric and gastric sleeve procedures may reduce the incidence of GERD. An IMU on the device may communicate with the monitor to facilitate this.

The device may determine a limitation of motion associated with the surgical instrument based on a downstream positional configuration of the downstream positional configurations. The device may determine an alternative positional configuration that overcomes the limitation of motion. The device may generate a display notification that displays the alternative positional configuration.

The device may analyze a placement of a trocar relative to the patient orientation and the orientation of the surgical instrument. The device may correlate the determined orientation and the placement of the trocar with a characteristic of the surgical instrument. The device may calculate, based on the correlation, an access limitation for the surgical instrument associated with performing the next surgical step. The access limitation may be based on the placement of the trocar, and the positional configurations may be adjusted based on the access limitation.

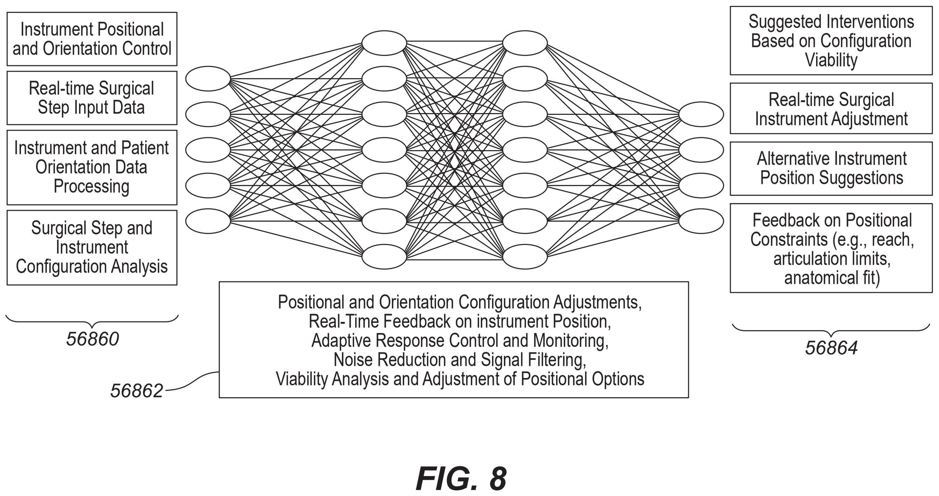

FIG. 8 illustrates an example artificial intelligence/machine learning flowchart associated with how the system may receive input, process data, and produce output related to a surgical procedure. At 56860, the system may receive instrument positional and orientation control data to track the location and orientation of surgical tools, such as the laparoscopic grasper, endoscope, and stapler. The system may receive real-time surgical step input data, including the procedural context of the active surgical step, patient anatomical variability, and previously executed steps. Instrument and patient orientation data processing may include the anatomical fit and positional constraints of surgical tools in real-time. The input data may include updates during the procedure to adapt to changes in the surgical site, tool configurations, and patient positioning.

At 56862, the system may analyze the surgical step and instrument configuration by processing the received input to determine viable positional and orientation configurations for surgical instruments. This may include modeling constraints such as reach limitations, articulation range, and anatomical fit, so that tools are aligned with current and downstream surgical requirements. Instrument and patient orientation data processing may correlate patient-specific anatomy with stored procedural datasets to refine instrument placement strategies and resolve potential conflicts in the configuration. Noise reduction and signal filtering may remove extraneous data, allowing the machine learning algorithms to focus on relevant inputs and provide actionable output.

In examples of instrument orientation control or advice to create a desired surgical effect, adjustment or display of options of positional or orientation implications on surgical tool usage may be implemented. Controlled movement or display notification of available options may be based on a present situation, an orientation, and/or a next surgical step or task.

At 56864, the system may generate output focused on positional and orientation configuration adjustments and provide feedback on instrument position to guide surgical precision. The output may include suggested interventions based on configuration viability, identifying unviable configurations and offering alternative configurations for instrument placement or orientation. For example, if stapler 56808 encounters an articulation limit while attempting to secure tissue near tumor 56810, the system may recommend adjusting instrument placement or repositioning the patient to improve access. The system may deliver surgical instrument adjustment suggestions, so that tools remain within operating ranges and maintain procedural flow.

The output at 56864 may include alternative instrument position suggestions, for example, in association with maintaining surgical workflow and maintaining alignment with the target anatomy. Feedback may include real-time overlays on a surgical display, highlighting constraints such as positional constraints (e.g., reach, articulation limits, anatomical fit) that may hinder procedural success. Adaptive response control and monitoring may adjust the system's recommendations based on updated input data or procedural progress, enabling the surgical team to respond (e.g., as needed).

A limitation of motion or presentation of the options may show the resulting anatomy or physiologic shape as part of the operation. Alternative options may be displayed, enabling the user to choose an option that fits a desired outcome. Choices that are not viable may be shown, and the cause of the non-viability may be highlighted (e.g., one or more of a reach, articulation range, port utilization, port placement, anatomic shape or thickness, previous surgical intervention or step).
The following list of numbered Examples forms part of the present disclosure:
1. A surgical system comprising:
   a processor configured to:
   identify an active surgical step and a subsequent surgical step associated with a surgical instrument;
   determine a plurality of positional configurations associated with the active surgical step;
   determine an orientation of the surgical instrument and a patient orientation associated with the active surgical step;
   determine a plurality of downstream positional configurations of the surgical instrument for performing the subsequent surgical step that correspond to the plurality of positional configurations associated with the active surgical step;
   adjust the plurality of positional configurations associated with the active surgical step based on the determined orientation of the surgical instrument, the patient orientation, and the determined plurality of downstream positional configurations of the surgical instrument for performing the next surgical step; and
   select a positional configuration from the adjusted plurality of positional configurations associated with the active surgical step.
2. The surgical system of Example 1, wherein the processor is further configured to:
   determine a plurality of anticipated effects based on the plurality of positional configurations associated with the active surgical step;
   generate a display notification configured to display the plurality of anticipated effects on an anatomic shape;
   receive an input based on the display notification, wherein the positional configuration is selected based on the received input; and
   generate a control signal configured to maneuver the surgical instrument to move in accordance with the selected positional configuration.
3. The surgical system of any of Example 1, wherein the processor is further configured to:
   determine a plurality of orientation configurations associated with the active surgical step;
   determine a plurality of downstream orientation options of the surgical instrument for performing the subsequent surgical step that correspond to the plurality of orientation options associated with the active surgical step;
   adjust the plurality of orientation options associated with the active surgical step based on the determined orientation of the surgical instrument, the patient orientation, and the determined plurality of downstream orientation options of the surgical instrument for performing the next surgical step; and
   select an orientation option from the adjusted plurality of orientation options associated with the active surgical step.
4. The surgical system of Example 3, wherein the processor is further configured to:
   determine a plurality of anticipated effects based on the plurality of orientation options associated with the active surgical step;
   generate a display notification configured to display the plurality of anticipated effects on an anatomic shape;
   receive an input based on the display notification, wherein the orientation option is selected based on the received input; and
   generate a control signal configured to maneuver the surgical instrument to move in accordance with the selected orientation option.
5. The surgical system of Example 1, wherein the processor is further configured to:
   determine that a positional configuration of the plurality of positional configurations is unviable based on the active surgical step and the next surgical step;
   identify a cause associated with the positional configuration being unviable, wherein the plurality of positional configurations is adjusted based on the positional configuration being unviable and the cause associated with the positional configuration being unviable; and
   generate a display notification that displays the cause associated with the positional configuration being unviable.
6. The surgical system of Example 5, wherein the cause comprises one or more of a reach, an articulation range, a port utilization, a port placement, an anatomic shape, an anatomic thickness, or a previous surgical step.
7. The surgical system of Example 1, wherein the processor is further configured to:
   determine a limitation of motion associated with the surgical instrument based on a downstream positional configuration of the plurality of downstream positional configurations;
   determine an alternative positional configuration that overcomes the limitation of motion; and
   generate a display notification that displays the alternative positional configuration.
8. The surgical system of Example 1, wherein the processor is further configured to:
   analyze a placement of a trocar relative to the patient orientation and the orientation of the surgical instrument;
   correlate the determined orientation and the placement of the trocar with a characteristic of the surgical instrument; and
   calculate, based on the correlation, an access limitation for the surgical instrument associated with performing the next surgical step, wherein the access limitation is based on the placement of the trocar, and wherein the plurality of positional configurations is adjusted based on the access limitation.
9. A method for a surgical system, the method comprising:
   identifying an active surgical step and a subsequent surgical step associated with a surgical instrument;
   determining a plurality of positional configurations associated with the active surgical step;
   determining an orientation of the surgical instrument and a patient orientation associated with the active surgical step;
   determining a plurality of downstream positional configurations of the surgical instrument for performing the subsequent surgical step that correspond to the plurality of positional configurations associated with the active surgical step;
   adjusting the plurality of positional configurations associated with the active surgical step based on the determined orientation of the surgical instrument, the patient orientation, and the determined plurality of downstream positional configurations of the surgical instrument for performing the next surgical step; and
   selecting a positional configuration from the adjusted plurality of positional configurations associated with the active surgical step.
10. The method of Example 9, wherein the method further comprises:
   determining a plurality of anticipated effects based on the plurality of positional configurations associated with the active surgical step;
   generating a display notification configured to display the plurality of anticipated effects on an anatomic shape;
   receiving an input based on the display notification, wherein the positional configuration is selected based on the received input; and
   generating a control signal configured to maneuver the surgical instrument to move in accordance with the selected positional configuration.
11. The method of any of Example 9, wherein the method further comprises:
   determining a plurality of orientation configurations associated with the active surgical step;
   determining a plurality of downstream orientation options of the surgical instrument for performing the subsequent surgical step that correspond to the plurality of orientation options associated with the active surgical step;
   adjusting the plurality of orientation options associated with the active surgical step based on the determined orientation of the surgical instrument, the patient orientation, and the determined plurality of downstream orientation options of the surgical instrument for performing the next surgical step; and
   selecting an orientation option from the adjusted plurality of orientation options associated with the active surgical step.
12. The method of Example 11, wherein the method further comprises:
   determining a plurality of anticipated effects based on the plurality of orientation options associated with the active surgical step;
   generating a display notification configured to display the plurality of anticipated effects on an anatomic shape;
   receiving an input based on the display notification, wherein the orientation option is selected based on the received input; and
   generating a control signal configured to maneuver the surgical instrument to move in accordance with the selected orientation option.
13. The method of Example 9, wherein the method further comprises:
   determining that a positional configuration of the plurality of positional configurations is unviable based on the active surgical step and the next surgical step;
   identifying a cause associated with the positional configuration being unviable, wherein the plurality of positional configurations is adjusted based on the positional configuration being unviable and the cause associated with the positional configuration being unviable; and
   generating a display notification that displays the cause associated with the positional configuration being unviable.
14. The method of Example 13, wherein the cause comprises one or more a reach, an articulation range, a port utilization, a port placement, an anatomic shape, an anatomic thickness, or a previous surgical step.
15. The method of Example 9, wherein the method further comprises:
   determining a limitation of motion associated with the surgical instrument based on a downstream positional configuration of the plurality of downstream positional configurations;
   determining an alternative positional configuration that overcomes the limitation of motion; and
   generating a display notification that displays the alternative positional configuration.
16. The method of Example 9, wherein the method further comprises:
   analyzing a placement of a trocar relative to the patient orientation and the orientation of the surgical instrument;
   correlating the determined orientation and the placement of the trocar with a characteristic of the surgical instrument; and
   calculating, based on the correlation, an access limitation for the surgical instrument associated with performing the next surgical step, wherein the access limitation is based on the placement of the trocar, and wherein the plurality of positional configurations is adjusted based on the access limitation.
17. A surgical system comprising:
   a processor configured to:
   identify an active surgical step and a subsequent surgical step associated with a surgical instrument;
   determine a plurality of positional configurations associated with the active surgical step;
   determine an orientation of the surgical instrument and a patient orientation associated with the active surgical step;
   determine a plurality of downstream positional configurations of the surgical instrument for performing the subsequent surgical step that correspond to the plurality of positional configurations associated with the active surgical step; and
   adjust the plurality of positional configurations associated with the active surgical step based on the determined orientation of the surgical instrument, the patient orientation, and the determined plurality of downstream positional configurations of the surgical instrument for performing the next surgical step.
18. The surgical system of Example 17, wherein the processor is further configured to:
   determine a plurality of anticipated effects based on the plurality of positional configurations associated with the active surgical step;
   generate a display notification configured to display the plurality of anticipated effects on an anatomic shape;
   select a positional configuration from the adjusted plurality of positional configurations associated with the active surgical step;
   receive an input based on the display notification, wherein the positional configuration is selected based on the received input; and
   generate a control signal configured to maneuver the surgical instrument to move in accordance with the selected positional configuration.
19. The surgical system of any of Example 17, wherein the processor is further configured to:
   determine a plurality of orientation configurations associated with the active surgical step;
   determine a plurality of downstream orientation options of the surgical instrument for performing the subsequent surgical step that correspond to the plurality of orientation options associated with the active surgical step;
   adjust the plurality of orientation options associated with the active surgical step based on the determined orientation of the surgical instrument, the patient orientation, and the determined plurality of downstream orientation options of the surgical instrument for performing the next surgical step; and
   select an orientation option from the adjusted plurality of orientation options associated with the active surgical step.
20. The surgical system of Example 19, wherein the processor is further configured to:
   determine a plurality of anticipated effects based on the plurality of orientation options associated with the active surgical step;
   generate a display notification configured to display the plurality of anticipated effects on an anatomic shape;
   receive an input based on the display notification, wherein the orientation option is selected based on the received input; and
   generate a control signal configured to maneuver the surgical instrument to move in accordance with the selected orientation option.

## Claims

1. A surgical system comprising:
a processor configured to:
identify an active surgical step and a subsequent surgical step associated with a surgical instrument;
determine a plurality of positional configurations associated with the active surgical step;
determine an orientation of the surgical instrument and a patient orientation associated with the active surgical step;
determine a plurality of downstream positional configurations of the surgical instrument for performing the subsequent surgical step that correspond to the plurality of positional configurations associated with the active surgical step;
adjust the plurality of positional configurations associated with the active surgical step based on the determined orientation of the surgical instrument, the patient orientation, and the determined plurality of downstream positional configurations of the surgical instrument for performing the subsequent surgical step; and
select a positional configuration from the adjusted plurality of positional configurations associated with the active surgical step.

2. A computer-readable medium comprising instructions which, when executed by a computer, cause the computer to:
identify an active surgical step and a subsequent surgical step associated with a surgical instrument;
determine a plurality of positional configurations associated with the active surgical step;
determine an orientation of the surgical instrument and a patient orientation associated with the active surgical step;
determine a plurality of downstream positional configurations of the surgical instrument for performing the subsequent surgical step that correspond to the plurality of positional configurations associated with the active surgical step;
adjust the plurality of positional configurations associated with the active surgical step based on the determined orientation of the surgical instrument, the patient orientation, and the determined plurality of downstream positional configurations of the surgical instrument for performing the subsequent surgical step; and
select a positional configuration from the adjusted plurality of positional configurations associated with the active surgical step.

3. The surgical system of claim 1 or computer-readable medium of claim 2, wherein the processor is further configured to, or the instructions further cause the computer to:
determine a plurality of anticipated effects based on the plurality of positional configurations associated with the active surgical step;
generate a display notification configured to display the plurality of anticipated effects on an anatomic shape;
receive an input based on the display notification, wherein the positional configuration is selected based on the received input; and
generate a control signal configured to maneuver the surgical instrument to move in accordance with the selected positional configuration.

4. The surgical system or computer-readable medium of any of claims 1 to 3, wherein the processor is further configured to, or the instructions further cause the computer to:
determine a plurality of orientation configurations associated with the active surgical step;
determine a plurality of downstream orientation options of the surgical instrument for performing the subsequent surgical step that correspond to the plurality of orientation options associated with the active surgical step;
adjust the plurality of orientation options associated with the active surgical step based on the determined orientation of the surgical instrument, the patient orientation, and the determined plurality of downstream orientation options of the surgical instrument for performing the subsequent surgical step; and
select an orientation option from the adjusted plurality of orientation options associated with the active surgical step.

5. The surgical system or computer-readable medium of claim 4, wherein the processor is further configured to, or the instructions further cause the computer to:
determine a plurality of anticipated effects based on the plurality of orientation options associated with the active surgical step;
generate a display notification configured to display the plurality of anticipated effects on an anatomic shape;
receive an input based on the display notification, wherein the orientation option is selected based on the received input; and
generate a control signal configured to maneuver the surgical instrument to move in accordance with the selected orientation option.

6. The surgical system or computer-readable medium of any of claims 1 to 5, wherein the processor is further configured to, or the instructions further cause the computer to:
determine that a positional configuration of the plurality of positional configurations is unviable based on the active surgical step and the subsequent surgical step;
identify a cause associated with the positional configuration being unviable, wherein the plurality of positional configurations is adjusted based on the positional configuration being unviable and the cause associated with the positional configuration being unviable; and
generate a display notification that displays the cause associated with the positional configuration being unviable.

7. The surgical system or computer-readable medium of claim 6, wherein the cause comprises one or more of a reach, an articulation range, a port utilization, a port placement, an anatomic shape, an anatomic thickness, or a previous surgical step.

8. The surgical system or computer-readable medium of any of claims 1 to 7, wherein the processor is further configured to, or the instructions further cause the computer to:
determine a limitation of motion associated with the surgical instrument based on a downstream positional configuration of the plurality of downstream positional configurations;
determine an alternative positional configuration that overcomes the limitation of motion; and
generate a display notification that displays the alternative positional configuration.

9. The surgical system or computer-readable medium of any of claims 1 to 8, wherein the processor is further configured to, or the instructions further cause the computer to:
analyze a placement of a trocar relative to the patient orientation and the orientation of the surgical instrument;
correlate the determined orientation and the placement of the trocar with a characteristic of the surgical instrument; and
calculate, based on the correlation, an access limitation for the surgical instrument associated with performing the subsequent surgical step, wherein the access limitation is based on the placement of the trocar, and wherein the plurality of positional configurations is adjusted based on the access limitation.

10. A surgical system comprising:
a processor configured to:
identify an active surgical step and a subsequent surgical step associated with a surgical instrument;
determine a plurality of positional configurations associated with the active surgical step;
determine an orientation of the surgical instrument and a patient orientation associated with the active surgical step;
determine a plurality of downstream positional configurations of the surgical instrument for performing the subsequent surgical step that correspond to the plurality of positional configurations associated with the active surgical step; and
adjust the plurality of positional configurations associated with the active surgical step based on the determined orientation of the surgical instrument, the patient orientation, and the determined plurality of downstream positional configurations of the surgical instrument for performing the subsequent surgical step.

11. A computer-readable medium comprising instructions which, when executed by a computer, cause the computer to:
identify an active surgical step and a subsequent surgical step associated with a surgical instrument;
determine a plurality of positional configurations associated with the active surgical step;
determine an orientation of the surgical instrument and a patient orientation associated with the active surgical step;
determine a plurality of downstream positional configurations of the surgical instrument for performing the subsequent surgical step that correspond to the plurality of positional configurations associated with the active surgical step; and
adjust the plurality of positional configurations associated with the active surgical step based on the determined orientation of the surgical instrument, the patient orientation, and the determined plurality of downstream positional configurations of the surgical instrument for performing the subsequent surgical step.

12. The surgical system of claim 10 or computer-readable medium of claim 11, wherein the processor is further configured to, or the instructions further cause the computer to:
determine a plurality of anticipated effects based on the plurality of positional configurations associated with the active surgical step;
generate a display notification configured to display the plurality of anticipated effects on an anatomic shape;
select a positional configuration from the adjusted plurality of positional configurations associated with the active surgical step;
receive an input based on the display notification, wherein the positional configuration is selected based on the received input; and
generate a control signal configured to maneuver the surgical instrument to move in accordance with the selected positional configuration.

13. The surgical system or computer-readable medium of any of claims 10 to 12, wherein the processor is further configured to, or the instructions further cause the computer to:
determine a plurality of orientation configurations associated with the active surgical step;
determine a plurality of downstream orientation options of the surgical instrument for performing the subsequent surgical step that correspond to the plurality of orientation options associated with the active surgical step;
adjust the plurality of orientation options associated with the active surgical step based on the determined orientation of the surgical instrument, the patient orientation, and the determined plurality of downstream orientation options of the surgical instrument for performing the subsequent surgical step; and
select an orientation option from the adjusted plurality of orientation options associated with the active surgical step.

14. The surgical system or computer-readable medium of claim 13, wherein the processor is further configured to, or the instructions further cause the computer to:
determine a plurality of anticipated effects based on the plurality of orientation options associated with the active surgical step;
generate a display notification configured to display the plurality of anticipated effects on an anatomic shape;
receive an input based on the display notification, wherein the orientation option is selected based on the received input; and
generate a control signal configured to maneuver the surgical instrument to move in accordance with the selected orientation option.
